# EUROPEAN PATENT APPLICATION

(11) **EP 4 631 417 A1**
(43) Date of publication of application: **15.10.2025**
(21) Application number: 23901001.0
(22) Date of filing: 29.11.2023
(51) Int. Cl.: A61B 1/247, A61B 1/00

(54) **DENTAL AND MEDICAL MIRROR WITHOUT SCREW LOOSENING**

(30) Priority: 05.12.2022 KR 20220167982
(71) Applicant: Denflex Co., Ltd., Seoul 08381 (KR)
(72) Inventor: KIM, Hyung-Woo, Goyang-si Gyeonggi-do 10374 (KR)
(74) Representative: Lermer, Christoph
(86) International application number: PCT/KR2023/019434
(87) International publication number: WO 2024/122982

(57) **Abstract**

Proposed is a dental and medical mirror used at dentist clinics and hospitals. The mirror includes a mirror body part gripped by the hand during use, and a mirror holder part which is coupled to the mirror body part, and has a mirror provided at one end thereof and is replaced after use for a predetermined period, and employs elastic coupling instead of conventional screw-coupling as the form of coupling of the mirror body part and the mirror holder part, thereby enabling the mirror body part and the mirror holder part to be easily and firmly coupled to each other by a simple fitting operation, and fully solving the problem of screw loosening occurring during a procedure in which the mirror is used.

## Description

### Technical Field

The present disclosure relates to a dental and medical mirror used at dentist clinics and hospitals and, more particularly, to a dental and medical mirror including: a mirror body part gripped by the hand during use; and a mirror holder part which is coupled to the mirror body part, and has a mirror provided at one end thereof and is replaced after use for a predetermined period, and employing elastic coupling instead of conventional screw-coupling as the form of coupling of the mirror body part and the mirror holder part, thereby enabling the mirror body part and the mirror holder part to be easily and firmly coupled to each other by a simple fitting operation, and fully solving the problem of screw loosening occurring during a procedure in which the mirror is used.

### Background Art

In general, a dental and medical mirror is a tool used to identify medical problems or examine the inside of the mouth, and consists of a mirror body gripped by the hand during use and a mirror holder having a mirror mounted on one end thereof, with the other end configured to be coupled to the mirror body by screw fastening. The dental and medical mirror is mainly inserted into the oral cavity to reflect light onto dark areas or to retract the soft tissues such as the tongue or cheek.

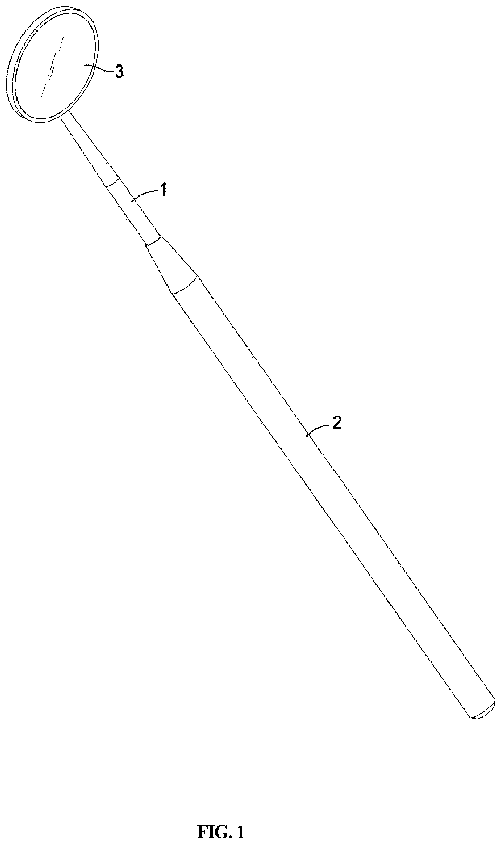

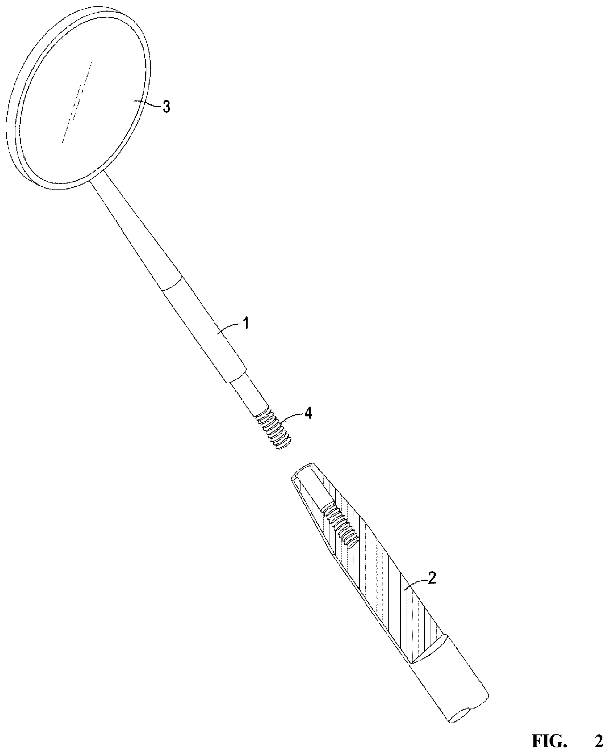

Referring to FIGS. 1 and 2, a conventional dental and medical mirror is composed of a mirror holder 1 equipped with a mirror 3 at one end thereof, and a mirror body 2 formed in a bar shape so that the practitioner can easily hold the body by hand when using the mirror. An axial hole is formed in the longitudinal direction at one end of the mirror body 2, and one end of the mirror holder 1 is configured to be inserted and coupled into the axial hole of the mirror body 2. A screw thread is formed on the interior surface of a predetermined portion of the axial hole of the mirror body 2, and a corresponding screw thread is formed on one end of the mirror holder 1 inserted into the shaft hole of the mirror body 2. Accordingly, the mirror holder 1 and the mirror body 2 have a structure that can be joined to each other through a screw-shaped joining part 4 by a screw fastening method, as shown in FIG. 2.

However, in the case of conventional dental and medical mirrors, the screw-shaped joining part that connects the mirror body and the mirror holder often comes loose unexpectedly during the procedure, especially due to the repeated movement of retracting the soft tissues such as the cheek or tongue, which causes inconvenience in having to stop the procedure and tighten the joining part again to firmly connect the mirror body and the mirror holder before reuse, and in turn, immediately decreases concentration of medical practitioners who need to focus on treatment and examination.

### Disclosure

### Technical Problem

An objective of the present disclosure is to provide a dental and medical mirror in which a mirror body part and a mirror holder part can be easily and firmly joined together by a simple fitting operation, and that can be easily disassembled (uncoupled, separated) if necessary, but the coupling of the hand-held mirror body part and the mirror holder part is never loosened to result in disassembly.

### Technical Solution

In order to achieve the above mentioned objective, there is provided a dental and medical mirror including: a mirror body part configured to have a longitudinal axial hole formed at an end thereof, a C-shaped spring receiving part in which a C-shaped spring can be positioned on an outer surface of a side where the axial hole is formed, and at least one fastener receiving side through-hole at a predetermined position of the C-shaped spring receiving part by penetrating from an outer surface of the mirror body part to the axial hole so that a fastener is positioned; the fastener positioned in the fastener receiving side through-hole; the C-shaped spring positioned in the C-shaped spring receiving part, and configured to have at least one first fastener mounting part at a position corresponding to the fastener receiving side through-hole; and a mirror holder part configured to have a mirror at a first end thereof and a fastening part including at least one second fastener mounting part on an outer surface of a second end thereof inserted into the axial hole of the mirror body part.

Preferably, the fastening part may further include an axial hole insertion guide part, and the axial hole insertion guide part may enable the fastening part of the mirror holder part to avoid entry resistance caused by the fastener protruding toward the axial hole when entering the axial hole of the mirror body part.

More preferably, the axial hole insertion guide part may be provided in the form of a recessed groove or flat surface from an end of the fastening part to at least a same position on a circumference where the second fastener mounting part is provided.

Preferably, the C-shaped spring receiving part may be provided in the form of a recessed groove along a circumference of the mirror body part on the outer surface of the side where the axial hole is formed.

More preferably, the first and second fastener mounting parts may be provided as recessed grooves or through holes capable of receiving and supporting the fastener.

Preferably, at least one anti-slip part recessed toward a longitudinal vertical central axis may be provided on the outer surface of the mirror body part adjacent to the C-shaped spring receiving part to prevent slipping when attaching a mirror part or using a mirror.

More preferably, the anti-slip part is provided in three recessed grooves or flat surfaces along a circumference of the mirror body part.

### Advantageous Effects

According to the present disclosure, the mirror holder part and the mirror body part can be easily and firmly joined together through a simple fitting operation, and can also be easily disassembled if necessary. Due to the strong and characteristic bonding structure, the hand-held mirror body part and the mirror holder part are never loosened or disassembled, especially during a procedure involving repeated action of retracting the cheek or tongue with the mirror.

### Description of Drawings

FIG. 1 is a perspective view of a conventional dental and medical mirror.
FIG. 2 is a combined perspective view of a conventional dental and medical mirror.
FIG. 3 is a perspective view of a dental and medical mirror according to an embodiment of the present disclosure.
FIG. 4 is an exploded perspective view of a dental and medical mirror according to an embodiment of the present disclosure.
FIG. 5 is a combined perspective view of a dental and medical mirror according to an embodiment of the present disclosure.
FIGS. 6(a) and 6(b) are combined cross-sectional views sequentially illustrating a process in which a mirror holder part is inserted into a mirror body part in a dental and medical mirror according to an embodiment of the present disclosure.
FIGS. 7(a) and 7(b) are combined cross-sectional views sequentially illustrating a process of rotating and joining a mirror holder part relative to a mirror body part while the mirror holder part is inserted into the mirror body part in a dental and medical mirror according to an embodiment of the present disclosure.

### Mode for Invention

Hereinafter, preferred embodiments of the present disclosure will be described in more detail with reference to the drawings.

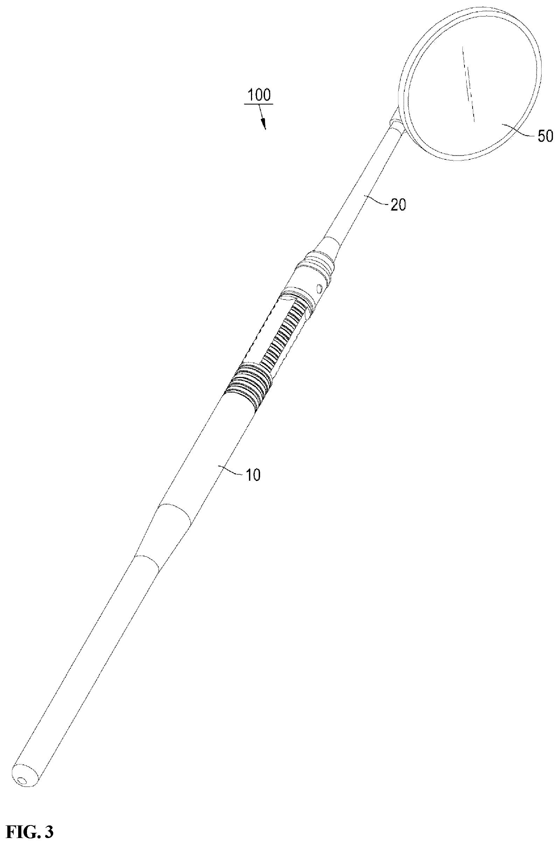

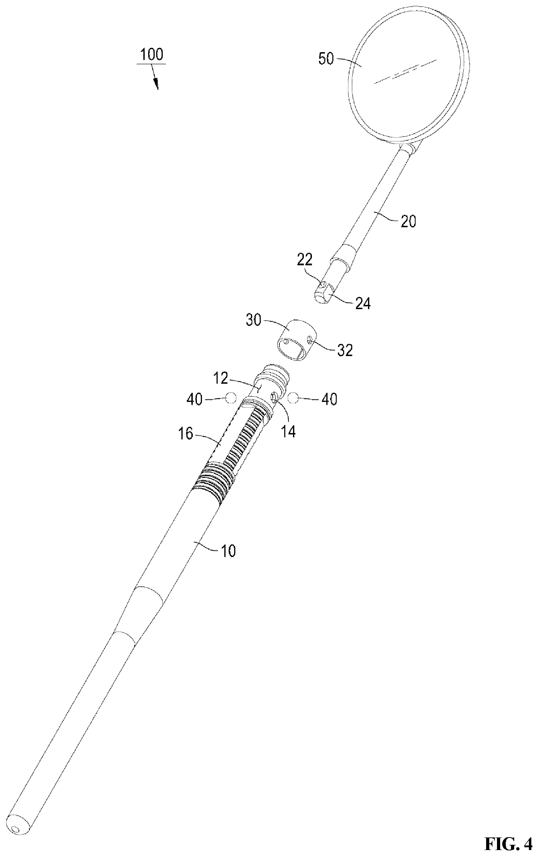

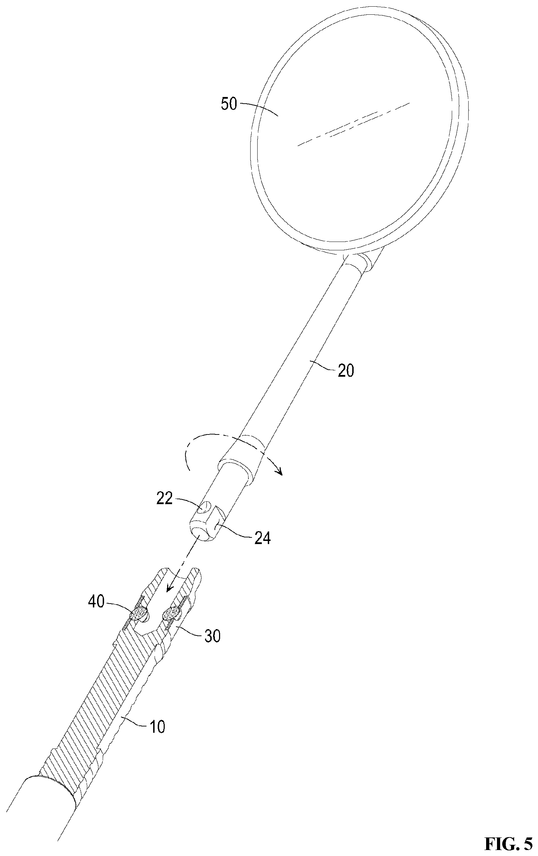

FIG. 3 is a perspective view of a dental and medical mirror according to an embodiment of the present disclosure, FIG. 4 is an exploded perspective view of a dental and medical mirror according to an embodiment of the present disclosure, and FIG. 5 is a combined perspective view of a dental and medical mirror according to an embodiment of the present disclosure.

Referring to FIGS. 3 to 5, a dental and medical mirror 100 according to an embodiment of the present disclosure consists of: a mirror body part 10 that can be held by hand by a practitioner and has a longitudinal axial hole formed at one end thereof; and a mirror holder part 20 having a mirror 30 at one end thereof and a fastening part at the other end thereof that is inserted into the axial hole of the mirror body part.

The mirror body part 10 is provided with a C-shaped spring receiving part 12 on the outer surface of one side where the axial hole is formed, in which a C-shaped spring 30 may be positioned. Preferably, the C-shaped spring receiving part 12 is formed in a recessed groove shape along the circumference of the mirror body part 10 on the outer surface of one side of the mirror body part where the axial hole is formed, as shown in FIG. 4. As shown in FIG. 4, at least one fastener receiving side through-hole 14 is formed at a predetermined position of the C-shaped spring receiving part 12 to pass through from the outer surface of the mirror body part 10 to the axial hole.

As shown in FIG. 5, a fastener 40 is positioned in the fastener receiving side through-hole 14. As shown in FIGS. 4 and 5, the C-shaped spring 30 is positioned in the C- shaped spring receiving part 12 of the mirror body part 10 and has at least one first fastener mounting part 32 at a position corresponding to the fastener receiving side through-hole 14. Preferably, the first fastener mounting part 32 of the C-shaped spring 30 is provided in the form of a recessed groove or through hole capable of receiving and supporting the fastener 40.

The fastener 40 is positioned between the fastener receiving side through-hole 14 of the mirror body part 10 and the first fastener mounting part 32 of the C-shaped spring 30, and as a result, the fastener 40 mounted in the fastener receiving side through-hole 14 is pressed toward the axial hole by the elastic action of the C-shaped spring 30.

The mirror holder part 20 is provided with the mirror 30 at one end thereof and the fastening part including at least one second fastener mounting part 22 on the outer surface of the other end thereof that is inserted into the axial hole of the mirror body part 10. Preferably, the second fastener mounting part 22 of the mirror holder part 20 is formed in a recessed groove or through hole capable of receiving and supporting the fastener 40. The fastening part further includes an axial hole insertion guide part 24 as shown in FIG. 4, and the axial hole insertion guide part 24 enables the fastening part of the mirror holder part 20 to avoid entry resistance caused by the fastener 40 protruding toward the axial hole when entering the axial hole of the mirror body part 10.

Preferably, the axial hole insertion guide part 24 is provided in the form of a recessed groove or flat surface from the end of the fastening part to at least the same position on the circumference where the second fastener mounting part 22 is formed.

Referring to FIG. 4, at least one anti-slip part 16 is provided on the outer surface adjacent to the C-shaped spring receiving part 12 to prevent slipping of the hand when attaching the mirror part or using the mirror. Preferably, the anti-slip part 16 is provided in at least two, more preferably three, recessed grooves or flat surfaces along the circumference of the mirror body part.

FIG. 5 is a combined perspective view of a dental and medical mirror according to an embodiment of the present disclosure. In particular, FIG. 5 illustrates a state immediately before the mirror body part 10 and the mirror holder part 20 of the dental and medical mirror of the present disclosure are combined. Referring to FIG. 5, the fastener 40 is positioned between the fastener receiving side through-hole 14 of the mirror body part 10 and the first fastener mounting part 32 of the C-shaped spring 30, and the fastener 40 maintained in a state in which the fastener 40 partially protrudes toward the axial hole by the elastic action of the C-shaped spring 30. To smoothly insert the fastening part of the mirror holder part 20 into the axial hole of the mirror body part 10 without resistance from the protruding fastener 40, the axial hole insertion guide part 24 provided et one end of the mirror holder part 20 needs to be inserted in a direction corresponding to the fastener 40 protruding toward the axial hole. For this reason, the axial hole insertion guide part 24 has the function of guiding the insertion direction of the mirror holder part 20 so as to avoid entry resistance caused by the fastener 40 protruding toward the axial hole when the fastening part of the mirror holder part 20 enters the axial hole of the mirror body part 10.

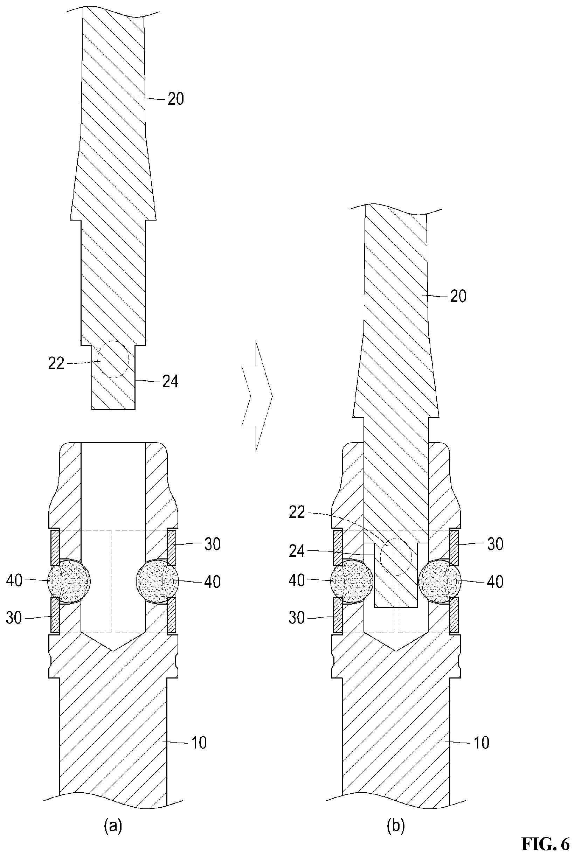

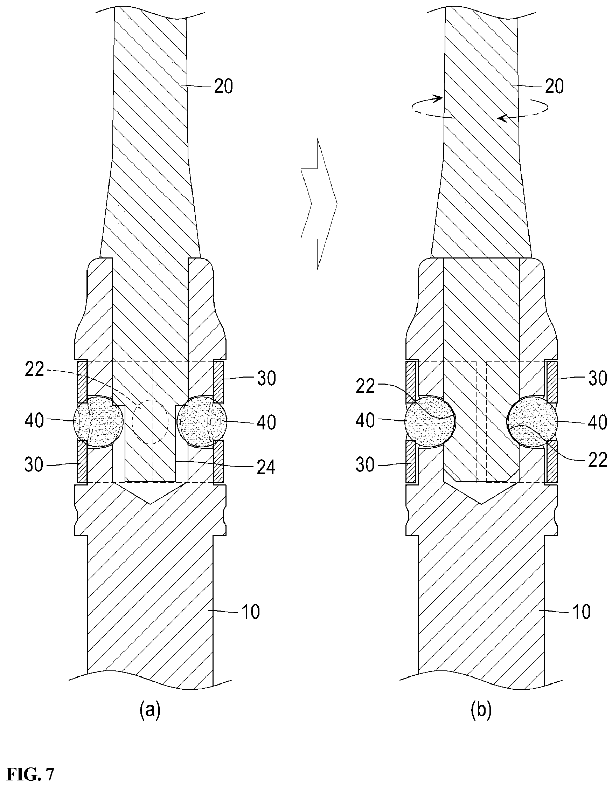

FIGS. 6(a) and 6(b) are combined cross-sectional views sequentially illustrating a process in which the mirror holder part is inserted into the mirror body part in the dental and medical mirror according to an embodiment of the present disclosure, and FIGS. 7(a) and 7(b) are combined cross-sectional views sequentially illustrating a process of rotating and joining the mirror holder part relative to the mirror body part while the mirror holder part is inserted into the mirror body part in the dental and medical mirror according to an embodiment of the present disclosure.

FIG. 6(a) is a view showing a state before insertion, and FIG. 6(b) is a view showing an intermediate process in which the mirror holder part 20 is partially inserted into the mirror body part 10. Referring to FIG. FIG. 6(b), when the axial hole insertion guide part 24 provided et one end of the mirror holder part 20 is inserted in a direction corresponding to the fastener 40 protruding toward the axial hole, due to the structural features of the axial hole insertion guide part 24 provided in the form of a recessed groove or flat surface, axial hole insertion guide part 24 may be smoothly inserted without any entry resistance due to the fastener 40 protruding toward the axial hole.

FIG. 7(a) is a view showing a state in which the mirror holder part 20 is fully inserted into the mirror body part 10. In this way, when the mirror holder part 20 is fully inserted into the mirror body part 10, and the mirror holder part 20 is slowly rotated relative to the mirror body part 10 (or rotating the mirror body part 10 relative to the mirror holder part 20 has the same effect) as shown in FIG. 7(b), the fastener 40 is mounted on the second fastener mounting part 22 of the mirror holder part 20 at a specific position where the second fastener mounting part 22 of the mirror holder part 20 and the fastener 40 correspond to each other.. As a result, a strong elastic coupling between the mirror body part 10 and the mirror holder part 20 is completed by means of the fastener 40 that is pressed by the elastic action of the C-shaped spring.

Although the present disclosure has been described above in detail with reference to the drawings with respect to specific embodiments, the present disclosure is not limited to such specific structures. Anyone with ordinary knowledge in the art will be able to modify or change the present disclosure in various ways without departing from the technical idea and scope of rights of the present disclosure described in the following patent claims. However, it should be made clear in advance that all such simple design material modifications or changing structures are clearly within the scope of the present disclosure.

## Claims

1. A dental and medical mirror used for oral examination and treatment, the mirror comprising:
a mirror body part configured to have a longitudinal axial hole formed at an end thereof, a C-shaped spring receiving part in which a C-shaped spring can be positioned on an outer surface of a side where the axial hole is formed, and at least one fastener receiving side through-hole at a predetermined position of the C-shaped spring receiving part by penetrating from an outer surface of the mirror body part to the axial hole so that a fastener is positioned;
the fastener positioned in the fastener receiving side through-hole;
the C-shaped spring positioned in the C-shaped spring receiving part, and configured to have at least one first fastener mounting part at a position corresponding to the fastener receiving side through-hole; and
a mirror holder part configured to have a mirror at a first end thereof and a fastening part including at least one second fastener mounting part on an outer surface of a second end thereof inserted into the axial hole of the mirror body part.

2. The mirror of claim 1, wherein the fastening part further comprises an axial hole insertion guide part, and the axial hole insertion guide part enables the fastening part of the mirror holder part to avoid entry resistance caused by the fastener protruding toward the axial hole when entering the axial hole of the mirror body part.

3. The mirror of claim 2, wherein the axial hole insertion guide part is provided in a form of a recessed groove or flat surface from an end of the fastening part to at least a same position on a circumference where the second fastener mounting part is provided.

4. The mirror of claim 1, wherein the C-shaped spring receiving part is provided in a form of a recessed groove along a circumference of the mirror body part on the outer surface of the side where the axial hole is formed.

5. The mirror of claim 1, wherein the first and second fastener mounting parts are provided as recessed grooves or through holes capable of receiving and supporting the fastener.

6. The mirror of claim 1, wherein at least one anti-slip part recessed toward a longitudinal vertical central axis is provided on the outer surface of the mirror body part adjacent to the C-shaped spring receiving part to prevent slipping when attaching a mirror part or using a mirror.

7. The mirror of claim 6, wherein the anti-slip part is provided in three recessed grooves or flat surfaces along a circumference of the mirror body part.
